# EUROPEAN PATENT SPECIFICATION

(11) **EP 4 630 084 B1**
(45) Date of publication and mention of the grant of the patent: **30.09.2026**
(21) Application number: 23821928.1
(22) Date of filing: 06.12.2023
(51) Int. Cl.: A61M 11/00, B05B 17/06

(54) **NEBULISATION METHOD AND APPARATUS**
VERNEBELUNGSVERFAHREN UND -VORRICHTUNG
PROCÉDÉ ET APPAREIL DE NÉBULISATION

(30) Priority: 06.12.2022 GB 202218328
(43) Date of publication of application: 15.10.2025
(73) Proprietor: Acu-Flow Limited, Glasgow G2 5QY (GB)
(72) Inventor: WITTE, Christian, Glasgow G2 6AY (GB); PRITCHARD, John, Glasgow G2 6AY (GB); NAZARZADEH, Elijah, Glasgow G2 6AY (GB); MACDONALD, Elspeth, Glasgow G2 6AY (GB)
(74) Representative: Mewburn Ellis LLP
(86) International application number: PCT/EP2023/084564
(87) International publication number: WO 2024/121247

(56) References cited:
- WO-A1-2004/085079
- WO-A1-2016/075209
- WO-A1-2022/219623
- JP-A- 2008 104 974
- JP-A- 2015 013 234
- JP-A- H11 114 467
- JP-A- H11 207 224
- KING XI ET AL: "Ultrasonic Surface Acoustic Wave platform for targeted pulmonary delivery of nano drug vehicles", 2019 IEEE INTERNATIONAL ULTRASONICS SYMPOSIUM (IUS), IEEE, 6 October 2019 (2019-10-06), pages 699 - 701, XP033671112, DOI: 10.1109/ULTSYM.2019.8925726

## Description

### Field of the Invention

The present invention relates to methods and apparatus for nebulisation of suspension formulations. Such methods and apparatus are of particular, although not necessarily exclusive, interest for applications in the preparation of therapeutic agents suitable for delivery to subjects.

### Background

According to the World Health Organization (WHO) there are 262 million people suffering from asthma and 392 million people with chronic obstructive pulmonary disease (COPD), leading to 3.7 million deaths per year worldwide. Many millions also suffer with pulmonary infectious disease, cystic fibrosis, pulmonary hypertension and allergic rhinitis as well as other under-diagnosed chronic respiratory diseases. Estimates of the cost of treating patients with such lung diseases, including those caused by tuberculosis (TB), COPD, cystic fibrosis, pneumonia, asthma and smoking was Euro 380 billion per annum (according to the European Lung White Book of the European Respiratory Society http://www.erswhitebook.org/ [accessed 07 October 2022]).

Inhalation drug delivery is known as the best route for treatment of respiratory disorders. One of the major advantages of the inhalation pulmonary route is that it can be targeted directly to the lung, and indeed, effective delivery of medication has been shown to be crucially dependent upon the droplet size distribution within the aerosol of medicine [1]. In general, if the size is too small (< 0.5 µm), the droplet will be exhaled, whilst if the size is too large (> 9.0 µm), the droplet will be trapped in the upper respiratory tract or throat. It is considered that pulmonary drug delivery requires droplet size distribution of the aerosols with diameters between about 1 and 5 µm. To treat the nasal passages, droplets up to 100 µm may be required.

The liquid formulations for treatment of these disorders are dispersed in the form of aerosols for patients to inhale. Water-insoluble active pharmaceutical ingredients (API) can be formulated as suspended particles, known as suspension formulations. The suspension formulations are found to be challenging for nebulisation, especially for nebulisation with ultrasonic nebulisers [2] [3]. The ultrasonic nebulisers use a focused beam of ultrasonic waves that, when applied to liquid formulations, create a liquid cone that droplets detach from to create an aerosol. The particles are trapped at the bottom of the cone and cannot be dispersed.

Nebulisation of suspensions using mesh nebulizers is limited by the size of the mesh. Studies have shown that mesh nebulizers, passive or active, can nebulize suspension drugs including marketed budesonide (Pulmicort^{™}), much better compared to ultrasonic nebulizers, however the drug dose delivered remains low compared to the drug loaded into the device (<22%) [4] [5]. For instance, marketed budesonide suspensions have a mean particle size in the suspension of 3 - 4 µm [6] [7], whilst the mesh orifice size in mesh nebulisers is usually 2-3µm. Thus, the budesonide particles usually clog the orifices of the mesh preventing a significant portion of the drug suspension being delivered.

In general, aerosolisation of suspension formulations has proven to be challenging for different types of nebulisers, with studies showing a considerable variation in their performance as measured with particle size [8] [9]. Thus, in short, there is a significant potential for improving the delivery of drugs by preparing aerosol droplets carrying higher concentrations of suspended particles that may be particularly suitable, for example, for entering the appropriate tissue within the lung. Such a technique would enable new therapies and improve existing therapies, allowing the reduction of healthcare costs and the improvement of clinical outcomes.

JP H11 207224 A discloses a medical inhaler configured such that a droplet ejected from a first droplet ejection device is supplied onto a surface of a surface acoustic wave generating/propagating substrate of a second droplet ejection device.

JP 2008 104974 A discloses a surface acoustic wave atomizer having a vibrator comprising a piezoelectric material having a pair of comb-shaped electrodes formed, and generating a surface acoustic wave by applying a high frequency voltage to the comb-shaped electrodes; and a liquid supply means for supplying a liquid to the surface of the vibrator. The surface acoustic wave atomizer may be used as a medical atomizer.

WO 2016/075209 A1 discloses controlled actuation of a liquid suspension placed in an array of suitable cavities and excited by surface acoustic waves at suitable frequencies can provide nebulised droplets of the liquid suspension with a tight distribution of droplet size. The liquid may be a pure compound; a mixture of liquids; a solution of one or more solutes in a liquid solvent; a suspension of particles (solid, substantially solid or liquid) in a carrier liquid; a colloid; an emulsion; nanoparticles or a suspension of nanoparticles. The liquid may be a medicament.

The present invention has been devised in light of the above considerations.

### Summary of the Invention

The invention is defined in the appended independent claims. Preferred embodiments are matter of the dependent claims.

In a first aspect, the present invention provides a method for the preparation of nebulised droplets as defined in the independent claim 1.

In a second aspect, the present invention provides a system for the preparation of nebulised droplets as defined in the independent claim 12.

By ensuring that the suspension formulation is delivered to the acoustic wave interaction surface as a time series of isolated volumes, it is considered that this provides the advantage of reducing or avoiding an effect on the liquid in the dispenser, in which the non-soluble particles of the suspension formulation would otherwise tend to cluster due to acoustic coupling with the transducer.

The volume of each isolated volume depends to an extent on the scale of the system. However, it is considered that relevant effects will be seen even for volumes of up to 50 µL. However, it is considered that more significant effects are seen for smaller volumes for the isolated volumes - for example up to 40 µL, up to 30 µL, up to 20 µL, up to 10 µL, up to 5 µL, up to 4 µL, up to 3 µL, up to 2 µL, or up to 1 µL. For example, in some embodiments, the isolated volume of the suspension formulation in the time series of isolated volumes has an initial volume in the range 0.5 to 5.0 µL.

The device may comprise an array of cavities opening at the acoustic wave interaction surface for containing the suspension formulation. It is intended that the acoustic waves interact with the suspension formulation in the cavities to produce the nebulised droplets. The array of cavities can be formed in a substrate that is also the substrate for the acoustic wave transducer. Alternatively, the array of cavities can be formed in a separate superstrate substrate that is configured to be acoustically coupled to the acoustic wave transducer.

The acoustic wave transducer may be operable to general surface acoustic waves (SAWs) for example, but the present invention is not necessarily limited to this and other acoustic waves can be used. Accordingly, the acoustic wave transducer may be operable to generate and/or propagate acoustic modes selected from one or more of Rayleigh waves (i.e. SAWs), Lamb waves, bulk acoustic waves and thickness mode.

During the step of locating the suspension formulation at the acoustic wave interaction surface, each isolated volume of the suspension formulation may for example only be in direct contact with only one of the dispenser and acoustic wave interaction surface at any one time, or not in contact with either the dispenser or the acoustic wave interaction surface. In this way, the isolated volume itself should not allow acoustic coupling between the dispenser and acoustic wave interaction surface. Accordingly, there is preferably an air (or other gas) gap between each isolated volume during a dispensing operation.

An outlet of the dispenser may be separated from the acoustic wave interaction surface by a minimum distance of not less than 0.1 mm. Depending on the volume of the isolated volumes, this distance may be not less than 0.2 mm, not less than 0.3 mm, not less than 0.4 mm, not less than 0.5 mm, not less than 0.6 mm, not less than 0.8 mm, not less than 1 mm, not less than 1.5 mm, not less than 2 mm, not less than 3 mm, not less than 4 mm, not less than 5 mm, not less than 6 mm, not less than 7 mm, not less than 8 mm, not less than 9 mm, or not less than 10 mm.

A time interval between dispensing consecutive isolated volumes in the time series of isolated volumes may be variable. For example, this variability may be set according to the rate of loss of suspension from the system due to nebulisation. The method may include a step of determining a nebulisation rate and/or evaporation rate of the suspension formulation located at the interaction surface and setting the time interval and/or the volume of the isolated volumes based on the determination. Such a step of determining a nebulisation rate and/or evaporation rate and setting the time interval may be repeated.

Each isolated volume of the suspension formulation may be located at the same position on the acoustic wave interaction surface. This may assist in ensuring time-based repeatability of the nebulisation and therefore time-based consistency of the nebulised plume from the system.

The non-soluble particles may comprise an active pharmaceutical ingredient.

The non-soluble particles have a particle size distribution with a D90 of at most 50 µm. Although this depends to some extent on the intended use and function of the non-soluble particles, the non-soluble particles may have a particle size distribution with a D90 of at most 40 µm, at most 30 µm, at most 20 µm, at most 10 µm, at most 8 µm, at most 6 µm, at most 5 µm, at most 4 µm, or at most 3 µm.

The acoustic wave interaction surface may be a surface acoustic wave (SAW) transmission surface. Accordingly, the acoustic wave transducer may be a SAW transducer, and the acoustic waves may be SAWs. Other acoustic energy may be used.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
Fig. 1 shows a schematic view of a system for the preparation and analysis of nebulised droplets.
Fig. 2 shows a modified version of Fig. 1.
Fig.3 shows the result of API recovery from different parts of a system when a solution (containing 1% saline and 1.1 mg/ml Allura red AC) with (right hand bars) and without (left hand bars) 4.8 um polystyrene beads is nebulised in contact mode with a microcavity arrangement.
Fig. 4 shows the results for nebulisation in contact mode of a suspension investigating the fluorescence signal of 4.8 µm green fluorescent polystyrene beads after collection from ACI stages.
Fig. 5 shows the effect of changing the approach used for Fig. 4 in order to ensure that the dispensing needle is not in contact with the substrate surface and to carry out drip feed of the suspension onto the substrate surface for nebulisation.
Fig. 6 shows the APSD of a 1% NaCl solution containing 1.1 mg/ml ARAC nebulised using a continuous delivery (200 µl/min) of solution with the dispenser needle in contact with a microcavity array from which the solution is nebulised.
Figs. 7-11 show the measured fluorescence at the different stages of the ACI in view of the addition of polystyrene beads (4.8 µm beads for Fig. 8, 1.0 µm beads for Fig. 9, 0.5 µm beads for Fig. 10, 0.2 µm beads for Fig. 11 and 0.1 µm beads for Fig. 12) to the 1% NaCl solution to create monodisperse suspensions. These results were obtained by nebulisation of the suspension using a continuous delivery (200 µl/min) of suspension with the dispenser needle in contact with a microcavity array from which the suspension is nebulised.
Fig. 12 shows the mass distribution of 4.8 µm beads on the ACI stages when nebulised at low flow rates (each at 50 µl/min) in contact and non-contact modes.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

We first summarise the present specific disclosure. Suspension formulations for inhalation therapy are often required because the drug (for example a corticosteroid) has insufficient solubility in a physiologically acceptable solvent system to deliver an efficacious dose.. However, delivery of suspensions is often complicated due to incompatibility of inhaler system with suspension particle size (e.g. mesh nebulisers) or unwanted particle interactions as the result of the aerosolisation mechanism (e.g. ultrasound nebulisers). The present disclosure studies the nebulisation of suspension formulations using surface acoustic waves (SAWs), although the use of different acoustic energy is also contemplated in order to achieve the advantages of the invention. The study also considers the use of a liquid confining microstructure.

It is demonstrated it is possible to carry out the nebulisation of monodisperse suspensions with particle sizes from 4.8 µm to 0.1 µm and the disclosure also identifies certain factors that when adjusted improve aerosolisation. It is found for example that particle dispersity is influenced by acoustic energy transmission into the liquid dispensing system and liquid confining microstructure. Acoustic cavity effects drive particle aggregation for larger particles sizes larger than 0.5 µm. This resulted in poor delivery of suspensions into the particle size analysis equipment (in this case an Andersen Cascade Impactor).

It is found that decoupling the dispensing system and suspension formulation reservoir from the SAW propagation path and adjusting the dispense mode to a non-continuous transducer improves the delivery of larger of 4.8 µm particles by 250%.

We now set out some further background in order to better understand the present disclosure.

Inhalation drug delivery is known as a particularly useful route for treatment of respiratory disorders. Liquid formulations for treatment of such disorders are dispersed in the form of aerosols for patients to inhale. In some cases, aqueous based formulations are used that contain water-insoluble active pharmaceutical ingredients (APIs) in the form of suspended particles. These are known as suspension formulations.

The particle sizes in suspension formulations typically lie in the range 10⁻² - 10 µm. A suspension is ideally monodisperse but particle interaction and physical instability of the formulation can lead to heterodispersity. In particular, particle sizes and clusters of 10⁻² - 10 µm can cause challenges during drug loading and dosing. Suspensions of large particles are subject to sedimentation and flocculation. The user has to make sure particles are fully suspended and dispersed in the solution before loading to avoid incorrect dosing. Once loaded into the inhaler device the treatment time should be short to avoid sedimentation and coagulation within the device itself, which may otherwise lead to incorrect dosing.

As discussed above, nebulisation of suspensions is considered to be challenging, especially with ultrasonic nebulisers. When a focused beam of ultrasonic waves is applied to the formulation, a liquid cone is created from which droplets detach in form of an aerosol. Suspended particles become trapped at the base of the cone, preventing dispersion and reducing loading of particles into the aerosol droplets.

Nebulisation of suspensions using mesh nebulisers is also discussed above.

Accordingly, in general, aerosolisation of suspension formulations has proven to be very difficult for different type of nebulisers, with studies showing a considerable variation in their performance with particle size.

Embodiments of the invention are now described in view of experimental work carried out.

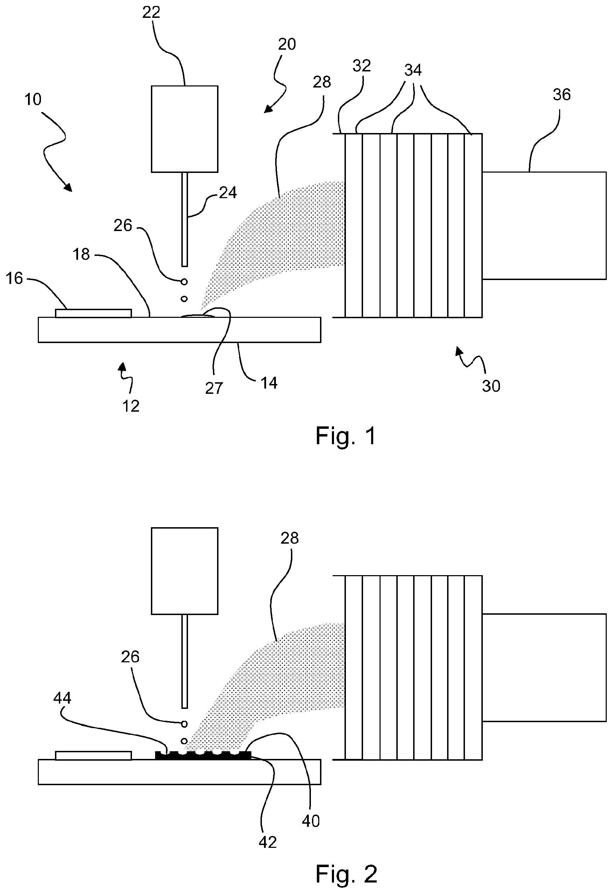

Figs. 1 and 2 each show a system for the preparation of nebulised droplets. Fig. 1 is described first and the relevant differences for the system of Fig. 2 are then described.

In the system of Fig. 1, there is a nebuliser device 10 comprising a transducer 12 having a piezoelectric material substrate 14 and an electrode arrangement 16, typically in the form of an interdigitated electrode arrangement. The electrode arrangement 16 is located on the top surface of the substrate 14 which in this embodiment is planar. There is also a region of the top surface of the substrate 14 where the electrode arrangement is not located. This region includes an acoustic wave interaction surface 18 onto which a suspension formulation is to be dispensed, as described later.

The system includes a dispenser 20 which comprises a suspension reservoir 22 and a dispensing conduit 24, which in this example is a needle. The dispenser may also include a pump (not shown) in order to cause flow of and meter the suspension from the dispenser.

The suspension formulation comprises a suspension of non-soluble particles in a carrier liquid.

In operation, a suitable electrical signal is applied to the electrode arrangement 16. In combination with the piezoelectric substrate 14, this forms a transducer that generates surface acoustic waves (SAWs) that propagate along the acoustic wave interaction surface 18. The SAWs interact with the suspension formulation located on the acoustic wave interaction surface 18 to produce nebulised droplets of the suspension formulation. The nebulised plume 28 is indicated schematically in Fig. 1.

The effect of having the end of the dispensing needle 24 spatially separated from the acoustic wave interaction surface 18, and not in contact with the transducer 12, is that the dispenser 22 is isolated from the SAWs propagating to and along the acoustic wave interaction surface 18. The present inventors have found that this prevents or reduces acoustic clustering of the non-soluble particles in the formulation in the dispenser.

In this embodiment, the drops 26 fall under gravity from the end of the dispensing needle onto the acoustic wave interaction surface 18. The effect of the SAWs propagating along the surface results in some acoustic streaming of the liquid along the surface, as shown schematically in Fig. 1 by the sessile drop 27 being located slightly offset from the position at which drops 26 impact the acoustic wave interaction surface 18. It will be understood that different modes of delivery of the suspension formulation to the acoustic wave interaction surface 18 are possible, for example by metering the isolated volumes of suspension formulation by spraying, slug flow or other equivalent approach.

The individual drops are isolated volumes of the suspension formulation and typically have consistent concentration of particles. The intention is that the rate of production of nebulised droplets (in volume terms) is the same as or similar to the rate of delivery of suspension formulation at the acoustic wave interaction surface 18. The transducer may be operated continuously or intermittently in order to achieve this, so that there is no substantial build-up of excess liquid at the acoustic wave interaction surface 18.

In order to carry out the experiments reported here, the nebulised plume 28 is drawn in a particle size analyser 30. In this example this is an Andersen Cascade Impactor with inlet 32, eight stages 34 of the cascade as described later and flow pump 36 to generate the air flow indicated in Fig. 1 as drawing the nebulised plume 28 towards and into the inlet 32.

Fig. 2 shows a similar arrangement to Fig. 1, identical features of which are not described again with respect to Fig. 2. The difference is that there is an microcavity arrangement 40 located on the surface of the substrate 14. This microcavity arrangement is an arrangement of wells (microcavities) 44 formed in the surface of a plate 42 of material, the plate of material being located on the upper surface of the substrate. In operation, the SAWs are coupled from the surface of the substrate into the plate of material. Based on earlier work, this is found to further improve the particle size distribution of the nebulised plume. The drops are directed to fall onto the microcavity arrangement and are nebulised from the microcavity arrangement.

Standard photolithography and metal lift-off processes were used to fabricate the surface acoustic wave device with interdigitated transducer design on 128° Y-cut lithium niobate piezoelectric substrate.

The microcavity array (also referred to herein as a microstructured chip) consisted of an array of wells and was fabricated by a dry etch process.

Nebulisation was performed with the microstructured chip placed on top of the SAW device.

A pump was used to dispense the suspension via a syringe needle onto the nebuliser device. The syringe needle tip was initially in contact with the surface of the nebuliser device (contact mode) but later adjusted into a non-contact mode configuration.

Monodisperse suspension formulation models were prepared using 1% (wt%) sodium chloride solution. Fluorescently labelled polystyrene particles were added to the solution (Table 1).

**Table 1. Monodisperse suspension formulation models. Polystyrene beads in 1% (wt%) sodium chloride aqueous solution.**

| **Particle size** | **Fluorescence** | **Concentration** | **Readout (Ex/Em)** | **Brand/Product number** |
|---|---|---|---|---|
| 4.8 um | Green | 1-2 mg/ml | 485/528 | Thermo Fisher^{™}/G0500 |
| 1.0 um | Red | 0.5 mg/ml | 580/605 | Thermo Fisher^{™}/R0100 |
| 0.5 um | Yellow-Green | 0.5 mg/ml | 485/528 | Invitrogen^{™}/F888 |
| 0.2 um | Yellow-Green | 0.5 mg/ml | 485/528 | Invitrogen^{™}/F8811 |
| 0.1 um | Red | 0.5 mg/ml | 580/605 | Invitrogen^{™}/F8801 |

The aerosol (nebulised plume) was characterised using an Anderson Cascade impactor (ACI). Size separated aerosol particles were collected from the ACI stages by rinsing with 5 ml of DI water. The fluorescent signal of the rinsate was analysed using a plate reader and signal values calculated as fold change to the blank signal of DI water for qualitative assessment of the nebulisation behaviour of suspensions.

A 1% (wt%) sodium chloride solution containing 1.1 mg/ml allura red ac (ARAC) dye and 2mg/ml 4.8 um fluorescence beads was used to study aerosol particle size distribution (APSD) during nebulisation of suspensions and the influence of its dispensing method. The dye concentration on each ACI stage was quantified using UV/VIS spectrometry (504 nm). The concentration of the 4.8 um particles was measured using the plate reader. Standard curves for dye and particle quantification were created prior to nebulisation.

The present inventors consider that particle aggregation can take place where significant volumes of the suspension are in direct contact with the piezoelectric substrate for extended periods of time. Furthermore, particle aggregation can also take place where the suspension is wicked into a porous matrix that is placed into continuous contact with the SAW substrate. Still further, particle aggregation takes place in larger liquid volumes in direct contact with the SAW substrate, e.g. large sessile droplets, where the action of travelling waves or standing waves within the liquid lead to particle aggregation at nodal pressure points.

The Andersen Cascade Impactor used in the present work operated to investigate particle size ranges in the different stages of the cascade, indicated in Table 2 as Stage 0 to Stage 7.

**Table 2: Particle cut off diameter for individual stages in Andersen Cascade Impactor**

| **Aerosol particle cut off diameter** | **Stage** |
|---|---|
| >9 µm | 0 |
| 5.8-9 µm | 1 |
| 4.7-5.8 µm | 2 |
| 3.3-4.7 µm | 3 |
| 2.1-3.3 µm | 4 |
| 1.1-2.1 µm | 5 |
| 0.7-1.1 µm | 6 |
| 0.4-0.7 µm | 7 |

The present disclosure shows that it is possible to reduce the suspension particle aggregation process and that this provides technical benefits to the ultrasound-based aerosol production. It is considered that these effects are made possible through a combination of controlled liquid dispensing rate, liquid dispensing method and liquid actuation towards aerosolization within a microstructure with cavities.

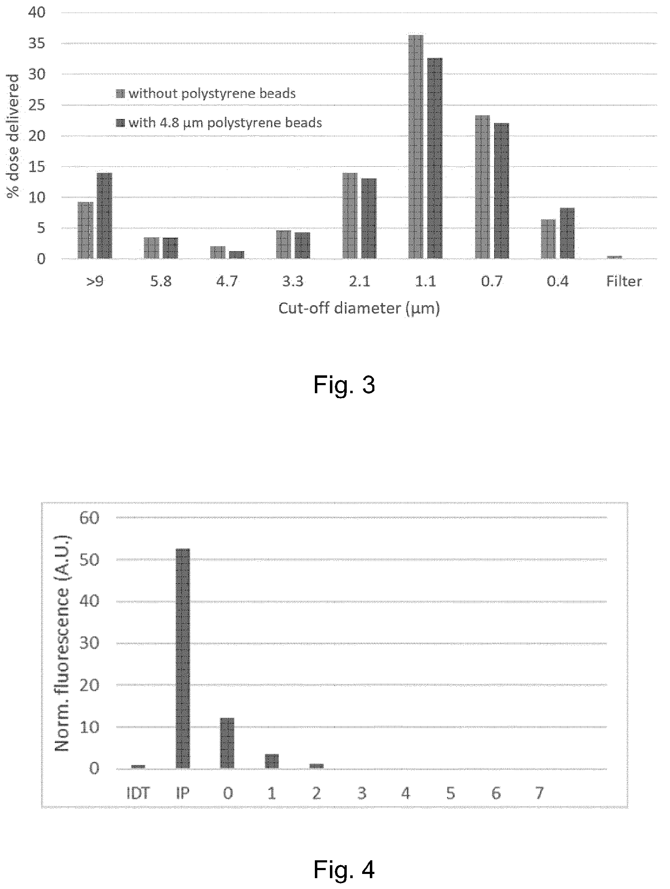

Fig. 3 shows the results of API recovery from different parts of the system when a solution with and without suspension particles is nebulised using the arrangement of Fig. 2. In Fig. 3, the amount of API recovered from different locations is shown, with the IDT being the surface of the transducer including the microcavity arrangement, the IP being the induction port of the cascade impactor and stages 0-7 being the stages of the cascade impactor as indicated in Table 2. In effect, Fig. 3 shows the aerodynamic particle size distribution (APSD) when nebulising 1% sodium chloride with and without suspension particles presence. The graph shows the amount of the recovered analyte (Allura Red AC) from each stage in the setup and ACI. IDT: SAW device, IP: Induction Port of ACI, numbers representing cut-off stages within the ACI and in accordance with Table 2. Nebulisation was carried out at 13.4 MHz, -5dBm, 200ul/min. The results for Fig. 3 are for the dispensing needle in contact with the SAW substrate and the microcavity arrangement. Nebulisation was carried out at 13.4 MHz, -5dBm, 200µl/min.

Fig. 4 shows the results for nebulisation of a suspension investigating the fluorescence signal of 4.8 µm green fluorescent polystyrene beads after collection from ACI stages. The results for Fig. 4 are for the dispensing needle in contact with the SAW substrate. Nebulisation at 13.4 MHz, -5dBm, 200µl/min.

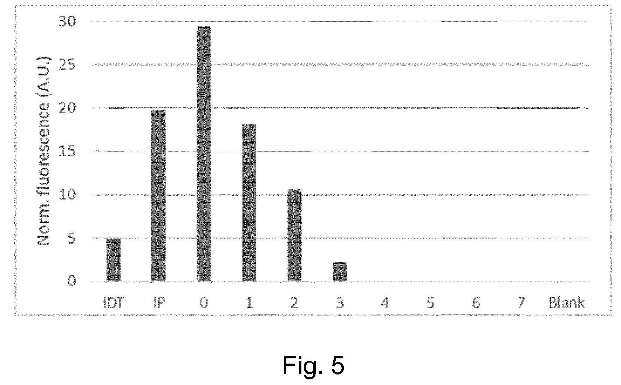

Fig. 5 shows the effect of changing the approach used for Fig. 4 in order to ensure that the dispensing needle is not in contact with the substrate surface and to carry out drip feed of the suspension onto the substrate surface for nebulisation. For Fig. 5, the suspension once again used is of 4.8 µm green fluorescent polystyrene beads but dispensed at 50 µl/min. Comparing Fig. 5 with Fig. 4 shows that the nebulised plume contains droplets of smaller particle size carrying the fluorescent marker.

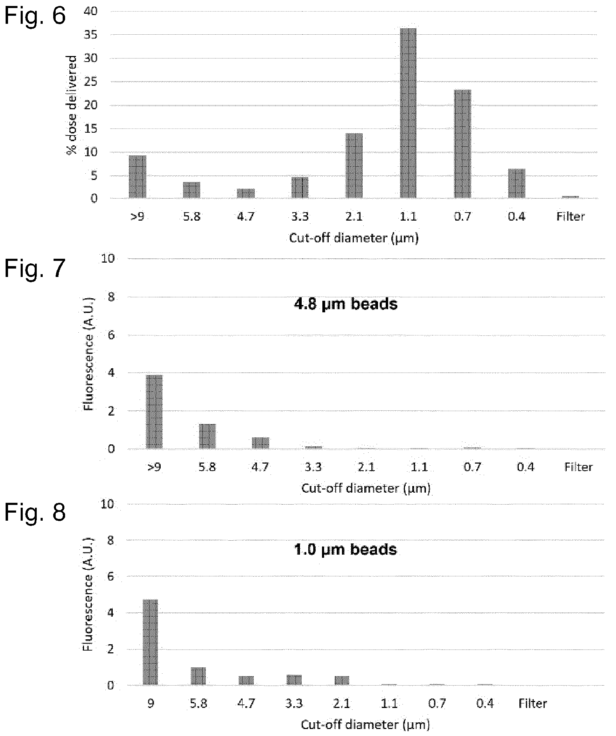

Further results are now explained with reference to Figs. 6-11.

The APSD of a 1% NaCl solution containing 1.1 mg/ml ARAC showed a typical bimodal distribution with peaks at 9 µm and 1.1 µm particle size (Fig. 6). This result is obtained by nebulisation of the solution using a continuous delivery (200 µl/min) of solution with the dispenser needle in contact with a microcavity array from which the solution is nebulised.

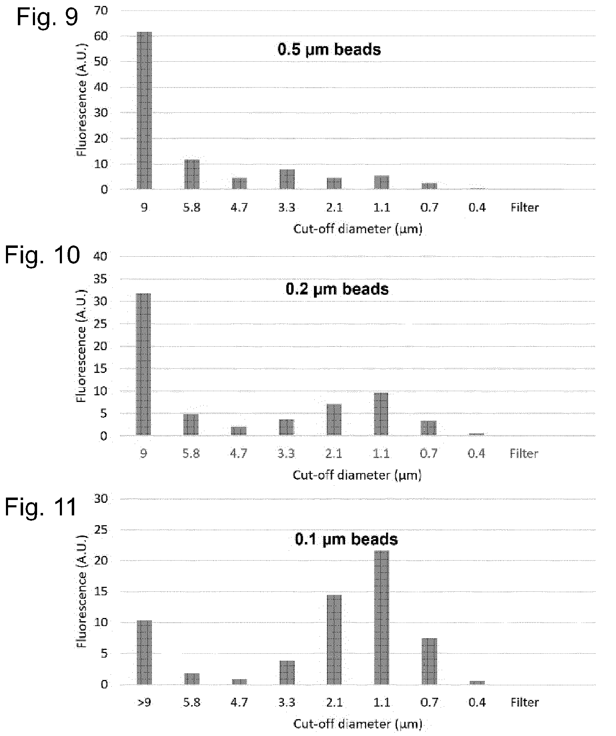

Figs. 7-11 show the measured fluorescence at the different stages of the ACI in view of the addition of polystyrene beads (4.8 µm beads for Fig. 7, 1.0 µm beads for Fig. 8, 0.5 µm beads for Fig. 9, 0.2 µm beads for Fig. 10 and 0.1 µm beads for Fig. 11) to the 1% NaCl solution to create monodisperse suspensions. These results were obtained by nebulisation of the suspension using a continuous delivery (200 µl/min) of suspension with the dispenser needle in contact with a microcavity array from which the suspension is nebulised.

The bead distributions within the ACI were distinct from the APSD shown in Fig. 6 in view of the absence of the bimodal distribution for suspensions containing 4.8 µm beads, 1 µm beads and 0.5 µm beads (Figs. 7-11). Only the suspensions with 0.2 µm and 0.1 µm beads (Figs. 10 and 11, respectively) showed a bimodal distribution. Additionally, it is noted that a large amount of beads accumulated on the nebuliser platform, with the microcavity array observed after the experiments showing a significant amount of beads aggregated within the microcavity array.

It is believed that acoustic cavity effects gave rise to acoustic forces that drive bead aggregation within the microcavity array and the dispensing needle tip. The continuous dispensing of the suspension into the microcavity array to produce a stable liquid film volume can support standing waves with pressure nodes ideal for bead aggregation. Additionally, coupling of acoustic energy into the continuous flow of the suspension mixture can concentrate beads and reduce dispersity prior to nebulisation process, resulting in formation of large aggregates on the platform (i.e. unsuccessful nebulisation).

Reduction of the flow rate (reduced from 200 µl/min to 50 µl/min) and avoiding direct exposure of the suspension mixture to the acoustic pressure were investigated to improve delivery of 4.8 µm beads into the ACI.

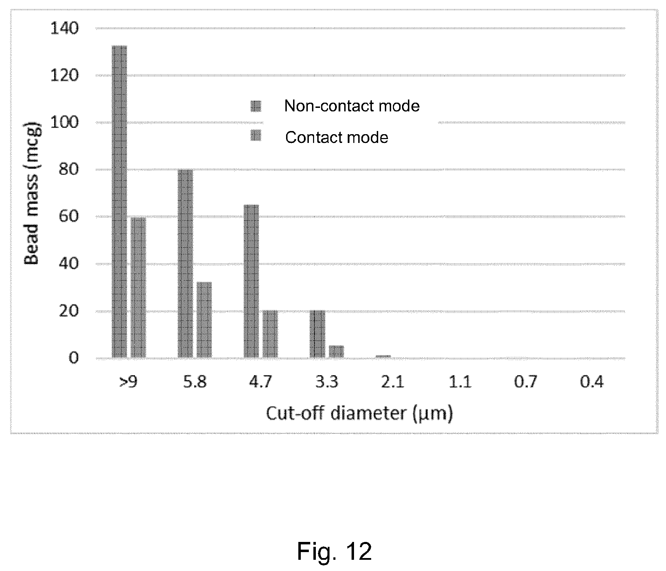

Fig. 12 compares the contact and non-contact approach to delivery of the suspension formulation to the microcavity array for nebulisation. In the contact mode, the needle is in continuous contact with the microcavity array and the liquid flows continuously to the microcavity array. In the non-contact mode, as in Fig. 2, the needle is separate from the microcavity array and the liquid is allowed to fall as free, isolated drops from the needle to the microcavity array.

The non-contact mode nebulisation of the liquid reduces the contact of the remaining bulk of the liquid in the reservoir and in the needle with the acoustic wave.

Fig. 12 shows the mass distribution of 4.8 µm beads on the ACI stages when nebulised at low flow rates (each at 50 µl/min) in contact and non-contact modes. In the non-contact mode, 300 µg of beads were collected across the >9 µm to 3.3 µm cut-off stages, while in contact mode 117 µg of beads were recovered. APSD data (not shown) for non-contact and contact mode showed an increase of 28% in mass of ARAC across the aforementioned stages, indicating a shift to larger droplet sizes which explains the increase by 250% in bead mass as shown in Fig. 12 only partially. The main contribution is believed to come from the change in dispensing method.

It is considered that the use of the microcavity array approach, combined with the controlled delivery rate and mode, disturbs particle aggregation phenomena and produces smaller droplet size. This is confirmed for example by Fig. 5.

In previous approaches, ultrasound-based nebulisers require direct contact, or indirect contact via a transmission medium, between the source of acoustic energy and the suspension formulation. Common devices actuate the whole liquid medication volume for aerosol creation. While this works for a single phase solution, a two phase solution containing insoluble (e.g. solid) particles in suspension are subjected to acoustic forces arising from the ultrasound waves. Here, acoustic radiation forces cause particles to accumulate at nodal pressure points within the liquid suspension volume or depending on the transmission medium at the solid liquid interface of the drug reservoir. The accumulation of drug particles to larger clusters prevents the formation of drug laden aerosol particles and hence causes insufficient dose delivery.

This phenomenon is also observed in SAW based actuation approaches where the suspension reservoir is in direct contact with the piezoelectric substrate [10]. In this study the dispensing needle is in contact with the actuated surface causing coupling of acoustic energy. The inner needle dimensions (160 µm diameter) are in favour of acting as acoustic cavity that support standing waves where particles undergo size dependent aggregation. In addition, the microstructure acts as cavity to confine liquid prior to nebulisation and can support standing waves that promote further particle clustering. Initial ACI experiments (Figs. 7-11) showed correlation between APSD and bead distribution for bead sizes below 0.5 µm. While it is not expected to see 4.8 µm beads in the lower cut-off stages, the lack of 1 µm and 0.5 µm beads indicates a change in droplet size formation, that corroborates the influence of bead size dependent acoustic radiation force.

The influence of acoustic forces is further highlighted by using contact and non-contact modes of nebulisation (i.e. adjusting the dispensing technique). The non-contact mode of liquid dispensation improves the delivery of 4.8 µm beads significantly. Compared to the contact mode (see Fig. 12), the non-contact mode reduces the effect of constant exposure of the liquid to the acoustic energy. The drip feeding through sequential droplet dispensing is believed to further reduce resonance behaviour due to the unsteady volume distribution in the cavities of the microcavity arrangement.

Accordingly, nebulisation of suspension formulations with ultrasound based transducer like SAW devices require careful consideration with regards to acoustic energy transmission into the liquid within the drug reservoirs and dispensing system. Particle clustering due to acoustic forces from travelling and standing waves prevents efficient aerosolization of suspensions. Decoupling of reservoirs and dispensing system from the actuated surface can reduce acoustic energy transmission and particle clustering prior to nebulisation. Thus, use of the non-contact mode for dispensing the formulation for nebulisation may prevent strong resonance effects that are associated with particle clustering.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention which is defined in the appended claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### References

A number of publications are cited above in order to more fully describe and disclose the invention and the state of the art to which the invention pertains. Full citations for these references are provided below.
[1] P. P. H. L. Brun, A. H. de Boer, H. W. Frijlink, and H. G. M. Heijerman, Pharm. World Sci., 2000, 22, 75-81.
[2] Nikander K, Turpeinen M, Wollmer P. "The Conventional Ultrasonic Nebulizer Proved Inefficient in Nebulizing a Suspension" J. Aero. Med. 1999 12:2, 47-53
[3] Pulmicort Respules Patient information, accessible here https://www.rxlist.com/pulmicort-respules-d rug.htm#description
[4] R.H.M. Hatley, J. Parker, L.E.A. Hardaker & S. Byrne. "Ultrasonic and Mesh Nebulizers Are Not the Same - Delivery of a Budesonide Suspension" Drug Delivery to the Lungs, DDL 2015
[5] L Slator, Y Degtyareva, R Potter & RHM Hatley. "Passive Mesh, Active Mesh and Ultrasonic Nebulizers - Ability to Deliver a Budesonide Suspension" Drug Delivery to the Lungs, DDL 2017
[6] H.S.M. Ali, P. York, A. Amani, N. Blagden, Evaluation of a Nanodispersion Formulation Prepared through Microfluidic Reactors for Pulmonary Delivery of Budesonide Using Nebulizers, Iran J Pharm Res, 13 (2014) 785-795.
[7] W.K. Kraft, B. Steiger, D. Beussink, J.N. Quiring, N. Fitzgerald, H.E. Greenberg, S.A. Waldman, The pharmacokinetics of nebulized nanocrystal budesonide suspension in healthy volunteers, J Clin Pharmacol, 44 (2004) 67-72.
[8] Najlah M, Parveen I, Alhnan MA, Ahmed W, Faheem A, Phoenix DA, Taylor KMG, Elhissi A. "The effects of suspension particle size on the performance of air-jet, ultrasonic and vibrating-mesh nebulisers". Int. J. Pharm. 2014, Vol 461, pp 234-241, https://doi.org/10.1016/j.ijpharm.2013.11.022.
[9] Angela Mary Fonceca, William Graham Fox Ditcham, Mark L. Everard, Sunalene Devadason, Drug Administration by Inhalation in Children, Editor(s): Robert William Wilmott, Robin Deterding, Albert Li, Felix Ratjen, Peter Sly, Heather J. Zar, Andrew Bush, Kendig's Disorders of the Respiratory Tract in Children (Ninth Edition), Elsevier, 2019, Pages 257-271.
[10] Witte C , Reboud J, Wilson R , Cooper J M, Neale S: Microfluidic resonant cavities enable acoustophoresis on a disposable superstrate, Lab Chip, 2014; 14: 4277 - 4283.

## Claims

1. A method for the preparation of nebulised droplets, including providing a device (10) having an acoustic wave interaction surface (18) and an acoustic wave transducer (16) adapted to generate and propagate acoustic waves to the acoustic wave interaction surface (18),
wherein the device further comprises a dispenser (20) arranged with respect to the acoustic wave interaction surface and configured to dispense a suspension formulation comprising a suspension of non-soluble particles in a carrier liquid,
the method including the steps:
locating the suspension formulation at the acoustic wave interaction surface in a time series of isolated volumes (26) using the dispenser, each isolated volume of suspension formulation having a volume of not more than 50 µL; and
causing acoustic waves to propagate to the acoustic wave interaction surface to interact with the suspension formulation to produce nebulised droplets (28) of the suspension formulation,
wherein, during the step of locating the suspension formulation at the acoustic wave interaction surface, the dispenser is isolated from acoustic waves propagating into the suspension formulation to prevent or reduce acoustic clustering of the non-soluble particles in the dispenser,
and wherein the non-soluble particles comprise an active pharmaceutical ingredient.

2. A method according to claim 1, wherein the device comprises an array of cavities (44) opening at the acoustic wave interaction surface for containing the suspension formulation, wherein the acoustic waves interact with the suspension formulation in the cavities to produce the nebulised droplets.

3. A method according to claims 1 or 2, wherein during the step of locating the suspension formulation at the acoustic wave interaction surface, each isolated volume of the suspension formulation is in direct contact with only one of the dispenser and acoustic wave interaction surface at any one time, or is not in contact with either the dispenser or the acoustic wave interaction surface.

4. A method according to claim 3, wherein an outlet of the dispenser is separated from the acoustic wave interaction surface by a minimum distance of not less than 0.1 mm.

5. A method according to any preceding claim, wherein a time interval between dispensing consecutive isolated volumes in the time series of isolated volumes is variable.

6. A method according to claim 5, including a step of determining a nebulisation rate and/or evaporation rate of the suspension formulation located at the interaction surface and setting the time interval and/or the volume of the isolated volumes based on the determination.

7. A method according to claim 6, wherein the step of determining a nebulisation rate and/or evaporation rate and setting the time interval is repeated.

8. A method according to any preceding claim, wherein each isolated volume of the suspension formulation in the time series of isolated volumes has an initial volume in the range 0.5 to 5.0 µL.

9. A method according to any preceding claim, wherein each isolated volume of the suspension formulation is located at the same position on the acoustic wave interaction surface.

10. A method according to any preceding claim, wherein the acoustic wave interaction surface is a surface acoustic wave (SAW) transmission surface, the acoustic wave transducer is a SAW transducer, and the acoustic waves are SAWs.

11. A method according to any preceding claim, wherein the non-soluble particles have a particle size distribution with a D90 of at most 50 µm.

12. A system for the preparation of nebulised droplets, the system comprising a nebuliser device (10) and a suspension formulation comprising a suspension of non-soluble particles in a carrier liquid, wherein the nebuliser device comprises:
an acoustic wave interaction surface (18);
an acoustic wave transducer (16) adapted to generate and propagate acoustic waves to the acoustic wave interaction surface;
a dispenser (20) configured to locate the suspension formulation at the acoustic wave interaction surface in a time series of isolated volumes (26), each isolated volume of suspension formulation having a volume of not more than 50 µL,
wherein, in operation, acoustic waves propagating to the acoustic wave interaction surface interact with the suspension formulation to produce nebulised droplets of the suspension formulation,
wherein the nebuliser device is configured to isolate the dispenser from the acoustic waves propagating to the acoustic wave interaction surface,
and wherein the non-soluble particles comprise an active pharmaceutical ingredient.

## Patentansprüche

1. Verfahren zur Herstellung vernebelter Tröpfchen, einschließlich des Bereitstellens einer Vorrichtung (10), die eine Wechselwirkungsoberfläche (18) akustischer Wellen und einen Wandler (16) akustischer Wellen aufweist, die ausgelegt ist, um akustische Wellen zu erzeugen und an die Wechselwirkungsoberfläche (18) akustischer Wellen auszubreiten,
wobei die Vorrichtung ferner ein Abgabeelement (20) umfasst, das in Bezug auf die Wechselwirkungsoberfläche akustischer Wellen angeordnet und ausgelegt ist, um eine Suspensionsformulierung, umfassend eine Suspension nichtlöslicher Partikel in einem Trägerfluid, abzugeben,
wobei das Verfahren folgende Schritte umfasst:
Positionieren der Suspensionsformulierung an der Wechselwirkungsoberfläche akustischer Wellen in einer Zeitreihe von isolierten Volumina (26) unter Verwendung des Abgabeelements, wobei jedes isolierte Volumen der Suspensionsformulierung ein Volumen von nicht mehr als 50 µl aufweist; und
Bewirken, dass sich akustische Wellen bis zur Wechselwirkungsoberfläche akustischer Wellen ausbreiten, um mit der Suspensionsformulierung wechselzuwirken, um vernebelte Tröpfchen (28) der Suspensionsformulierung zu erzeugen,
wobei während des Schritts des Positionierens der Suspensionsformulierung an der Wechselwirkungsoberfläche akustischer Wellen das Abgabeelement von akustischen Wellen, die sich in die Suspensionsformulierung ausbreiten, isoliert ist, um ein akustisches Clustering der nichtlöslichen Partikel in dem Abgabeelement zu verhindern oder zu reduzieren,
und wobei die nichtlöslichen Partikel einen pharmazeutischen Wirkstoff umfassen.

2. Verfahren nach Anspruch 1, wobei die Vorrichtung eine Anordnung von Hohlraum-(44) Öffnungen an der Wechselwirkungsoberfläche akustischer Wellen zum Enthalten der Suspensionsformulierung umfasst, wobei die akustischen Wellen mit der Suspensionsformulierung in den Hohlräumen wechselwirken, um die vernebelten Tröpfchen zu erzeugen.

3. Verfahren nach Anspruch 1 oder 2, wobei während des Schritts des Positionierens der Suspensionsformulierung an der Wechselwirkungsoberfläche akustischer Wellen jedes isolierte Volumen der Suspensionsformulierung nur mit einem aus dem Abgabeelement und der Wechselwirkungsoberfläche akustischer Wellen gleichzeitig in direktem Kontakt steht oder weder mit dem Abgabeelement noch mit der Wechselwirkungsoberfläche akustischer Wellen in Kontakt steht.

4. Verfahren nach Anspruch 3, wobei ein Auslass des Abgabeelements durch einen Mindestabstand von nicht weniger als 0,1 mm von der Wechselwirkungsoberfläche akustischer Wellen getrennt ist.

5. Verfahren nach einem der vorangegangenen Ansprüche, wobei ein Zeitabstand zwischen dem Abgeben aufeinanderfolgender isolierter Volumina in der Zeitreihe von isolierten Volumina variabel ist.

6. Verfahren nach Anspruch 5, einschließlich eines Schritts des Bestimmens einer Vernebelungsrate und/oder Verdampfungsrate der Suspensionsformulierung, die sich an der Wechselwirkungsoberfläche befinden, und des Einstellens des Zeitabstands und/oder des Volumens der isolierten Volumina basierend auf dem Bestimmen.

7. Verfahren nach Anspruch 6, wobei der Schritt des Bestimmens einer Vernebelungsrate und/oder einer Verdampfungsrate und des Einstellens des Zeitabstands wiederholt wird.

8. Verfahren nach einem der vorangegangenen Ansprüche, wobei jedes isolierte Volumen der Suspensionsformulierung in derselben Zeitreihe von isolierten Volumina ein Ausgangsvolumen im Bereich von 0,5 bis 5,0 µl aufweist.

9. Verfahren nach einem der vorangegangenen Ansprüche, wobei jedes isolierte Volumen der Suspensionsformulierung an derselben Position auf der Wechselwirkungsoberfläche akustischer Wellen angeordnet ist.

10. Verfahren nach einem der vorangegangenen Ansprüche, wobei die Wechselwirkungsoberfläche akustischer Wellen eine akustische Oberflächenwellen- (SAW-) Übertragungsoberfläche ist, der Wandler akustischer Wellen ein SAW-Wandler ist und die akustischen Wellen SAW sind.

11. Verfahren nach einem der vorangegangenen Ansprüche, wobei die nichtlöslichen Partikel eine Partikelgrößenverteilung mit einem D90 von höchstens 50 µm aufweisen.

12. System zur Herstellung vernebelter Tröpfchen, wobei das System eine Verneblervorrichtung (10) und eine Suspensionsformulierung, umfassend eine Suspension nichtlöslicher Partikel in einer Trägerflüssigkeit, umfasst, wobei die Verneblervorrichtung Folgendes umfasst:
eine Wechselwirkungsoberfläche (18) akustischer Wellen;
einen Wandler (16) akustischer Wellen, der ausgelegt ist, um akustische Wellen zu erzeugen und an die Wechselwirkungsoberfläche akustischer Wellen auszubreiten;
ein Abgabeelement (20), das ausgelegt ist, um die Suspensionsformulierung an der Wechselwirkungsoberfläche akustischer Wellen in einer Zeitreihe von isolierten Volumina (26) zu positionieren, wobei jedes isolierte Volumen der Suspensionsformulierung ein Volumen von nicht mehr als 50 µl aufweist,
wobei akustische Wellen, die sich bis zur Wechselwirkungsoberfläche akustischer Wellen ausbreiten, in Betrieb mit der Suspensionsformulierung wechselwirken, um vernebelte Tröpfchen der Suspensionsformulierung zu erzeugen,
wobei die Verneblervorrichtung ausgelegt ist, um das Abgabeelement von den akustischen Wellen, die sich bis zur Wechselwirkungsoberfläche akustischer Wellen ausbreiten, zu isolieren,
und wobei die nichtlöslichen Partikel einen pharmazeutischen Wirkstoff umfassen.

## Revendications

1. Procédé de préparation de gouttelettes nébulisées, comprenant la fourniture d'un dispositif (10) présentant une surface d'interaction d'ondes acoustiques (18) et un transducteur d'ondes acoustiques (16) adapté à générer et à propager des ondes acoustiques vers la surface d'interaction d'ondes acoustiques (18),
dans lequel le dispositif comprend en outre un distributeur (20) agencé par rapport à la surface d'interaction d'ondes acoustiques et configuré pour distribuer une formulation de suspension comprenant une suspension de particules non solubles dans un liquide porteur,
le procédé comprenant les étapes consistant à :
localiser la formulation de suspension au niveau de la surface d'interaction d'ondes acoustiques dans une série temporelle de volumes isolés (26) en utilisant le distributeur, chaque volume isolé de formulation de suspension présentant un volume ne dépassant pas 50 µL ; et
amener les ondes acoustiques à se propager vers la surface d'interaction d'ondes acoustiques pour interagir avec la formulation de suspension pour produire des gouttelettes nébulisées (28) de la formulation de suspension, dans lequel, au cours de l'étape de localisation de la formulation de suspension au niveau de la surface d'interaction d'ondes acoustiques, le distributeur est isolé des ondes acoustiques se propageant dans la formulation de suspension pour empêcher ou réduire le regroupement acoustique des particules non solubles dans le distributeur, et dans lequel les particules non solubles comprennent un ingrédient pharmaceutique actif.

2. Procédé selon la revendication 1, dans lequel le dispositif comprend un réseau de cavités (44) s'ouvrant au niveau de la surface d'interaction d'ondes acoustiques afin de contenir la formulation de suspension, dans lequel les ondes acoustiques interagissent avec la formulation de suspension dans les cavités pour produire les gouttelettes nébulisées.

3. Procédé selon la revendication 1 ou 2, dans lequel au cours de l'étape consistant à localiser la formulation de suspension au niveau de la surface d'interaction d'ondes acoustiques, chaque volume isolé de la formulation de suspension est en contact direct avec un seul élément parmi le distributeur et la surface d'interaction d'ondes acoustiques à un moment quelconque, ou n'est pas en contact avec le distributeur ni avec la surface d'interaction d'ondes acoustiques.

4. Procédé selon la revendication 3, dans lequel une sortie du distributeur est séparée de la surface d'interaction d'ondes acoustiques d'une distance minimale d'au moins 0,1 mm.

5. Procédé selon une quelconque revendication précédente, dans lequel un intervalle de temps entre la distribution de volumes isolés consécutifs dans la série temporelle de volumes isolés est variable.

6. Procédé selon la revendication 5, comprenant une étape consistant à déterminer un taux de nébulisation et/ou un taux d'évaporation de la formulation de suspension située au niveau de la surface d'interaction et à régler l'intervalle de temps et/ou le volume des volumes isolés sur la base de la détermination.

7. Procédé selon la revendication 6, dans lequel l'étape de détermination d'un taux de nébulisation et/ou d'un taux d'évaporation et de réglage de l'intervalle de temps est répétée.

8. Procédé selon une quelconque revendication précédente, dans lequel chaque volume isolé de la formulation de suspension dans la série temporelle de volumes isolés présente un volume initial dans la plage de 0,5 à 5,0 µL.

9. Procédé selon une quelconque revendication précédente, dans lequel chaque volume isolé de la formulation de suspension est situé au niveau de la même position sur la surface d'interaction d'ondes acoustiques.

10. Procédé selon une quelconque revendication précédente, dans lequel la surface d'interaction d'ondes acoustiques est une surface de transmission d'ondes acoustiques de surface (SAW), le transducteur d'ondes acoustiques est un transducteur SAW, et les ondes acoustiques sont des SAW.

11. Procédé selon une quelconque revendication précédente, dans lequel les particules non solubles présentent une distribution granulométrique avec un D90 ne dépassant pas 50 µm.

12. Système pour la préparation de gouttelettes nébulisées, le système comprenant un dispositif de nébulisation (10) et une formulation de suspension comprenant une suspension de particules non solubles dans un liquide porteur, dans lequel le dispositif de nébulisation comprend :
une surface d'interaction d'ondes acoustiques (18) ;
un transducteur d'ondes acoustiques (16) adapté à générer et à propager des ondes acoustiques vers la surface d'interaction d'ondes acoustiques ;
un distributeur (20) configuré pour localiser la formulation de suspension au niveau de la surface d'interaction d'ondes acoustiques dans une série temporelle de volumes isolés (26), chaque volume isolé de formulation de suspension présentant un volume ne dépassant pas 50 µL,
dans lequel, en fonctionnement, des ondes acoustiques se propageant vers la surface d'interaction d'ondes acoustiques interagissent avec la formulation de suspension pour produire des gouttelettes nébulisées de la formulation de suspension,
dans lequel le dispositif de nébulisation est configuré pour isoler le distributeur des ondes acoustiques se propageant vers la surface d'interaction d'ondes acoustiques, et
dans lequel les particules non solubles comprennent un ingrédient pharmaceutique actif.
